# EUROPEAN PATENT SPECIFICATION

(11) **EP 2 750 622 B1**
(45) Date of publication and mention of the grant of the patent: **30.12.2015**
(21) Application number: 12769729.0
(22) Date of filing: 10.08.2012
(51) Int. Cl.: A61C 1/08, A61C 13/00

(54) **METHOD FOR CALCULATING THE FINAL POSITION OF A DENTAL IMPLANT**
VERFAHREN ZUR BERECHNUNG DER ENDPOSITION EINES ZAHNIMPLANTATS
PROCÉDÉ POUR CALCULER LA POSITION FINALE D'UN IMPLANT DENTAIRE

(30) Priority: 25.10.2011 IT TO20110966
(43) Date of publication of application: 09.07.2014
(73) Proprietor: Safe Surgery S.r.l., 13900 Biella (IT)
(72) Inventor: Villa, Roberto, 13900 Biella (IT)
(74) Representative: Rondano, Davide
(86) International application number: PCT/IB2012/054090
(87) International publication number: WO 2013/061175

(56) References cited:
- FR-A1- 2 916 958
- US-A- 5 556 278
- US-A1- 2005 106 531
- US-A1- 2008 176 187

## Description

The present invention relates to a method for predicting the actual final positioning of a dental implant to be implanted in a patient's mouth in a hole made with the aid of a surgical template.

Software tools (such as those developed by the Materialise and NobelBiocare companies) for the digital planning of dental implant surgery have been available for a number of years. Starting from a CT scan performed on the patient while the patient wears a radiographic guide (on which a set of gutta percha markers have been positioned), the software allows to display the three-dimensional image of the patient's cranium (particularly the mandibular region, the maxillary region and the regions closest to these) on a computer, thus enabling the surgeon to make a preliminary examination of the bone structure (in respect of its thickness, shape and consistency), the position of the osseous canals in which the nerve bundles and the blood vessels run, and the condition of the remaining teeth. A CT scan is then performed on the radiographic guide only, after which the software is able to associate the patient's cranium with the radiographic guide in a precise way (using the gutta percha markers), and then to display on the computer the three-dimensional model of the assembly formed by the cranium and the radiographic guide. Using this three-dimensional model, the surgeon can simulate the positioning of the implants in advance, with the aim of making the best use of the patient's bone and avoiding the risk areas (nerves, blood vessels, maxillary sinuses, etc.). All this preliminary simulation and analysis can be carried out by the surgeon without involving the patient, and therefore in complete safety. When the operation has been fully planned, a surgical template is made and is positioned and fixed in the patient's mouth before the surgical operation in order to guide the drill (or drills) with which the surgeon has to make the holes for the implants. The surgical template is an object made of resin material in which, for each implant, and therefore for each hole to be made, a corresponding bushing is provided for receiving drill guide sleeves designed to guide drills of progressively greater diameter up to the diameter specified for the hole to be made, the bushing being positioned and orientated so as to define precisely the planned position and orientation for the hole into which the implant in question is to be inserted. As the surgeon has a surgical template in which the bushings are positioned and orientated to enable the holes to be made exactly where they were planned in the operation planning process, then all he has to do during the actual operation is to make the holes being guided by the drill guide sleeves inserted into the bushings of the surgical template positioned and fixed in the patient's mouth.

This method of operation planning is currently in widespread use in dental implant surgery, because it has the advantages of being minimally invasive, of involving a very low level of surgical risk, and of making it possible to explore bone regions which would otherwise be inaccessible and to use them in order to optimize the positioning of the implants. However, this method cannot currently be used with zygomatic implants, that is to say with dental implants intended to be fastened (screwed) to the zygoma bone. Zygomatic implants have the advantage of ensuring a very high success rate (95% to 100%) and of being able to immediately bear loads (because zygoma bone is typically a very compact and hard bone, and therefore provides high stability), thus allowing the surgeon to screw the teeth onto the implants within 24 hours after the operation. On the other hand, the insertion of a zygomatic implant requires to make very long holes (in the order of some centimetres) in a very delicate area of the cranium. Consequently, there is a risk of perforating the floor of the orbit, resulting in oedema and blindness, or damaging the zygomatic nerve, leading to lacrimation and palpebral pain. These risks, as well as the fact that the positioning of zygomatic implants is a complex and invasive operation, severely restrict the use of zygomatic implants on a wider scale. As mentioned above, the use of a surgical template produced by digital operation planning would greatly facilitate this type of operation, but is extremely hazardous in the case of zygomatic implants. Owing to the considerable length of these implants, the smallest errors in the positioning and/or orientation of the bushings can considerably alter the positions of the bottoms of the holes, running the risk of perforating the floor of the orbit or damaging blood vessels or nerves. The risk is in fact that, when the surgical template is positioned in the patient's mouth before the operation is started, it may not be exactly in the same position as the radiographic guide which was in the patient's mouth during the performance of the first CT scan (on the basis of which the operation was planned), because of the resilience of the soft tissues of the oral cavity on which the template rests, and consequently the position of the holes made with the aid of the surgical template may not coincide with that specified during the digital planning, resulting in a greater probability of injury to the patient. At the present time, therefore, the digital planning of zygomatic implant positioning operations does not ensure an adequate safety level.

The present invention was therefore devised primarily in order to enable surgical dental implantation operations to be performed with the use of zygomatic implants in conditions of greater safety, and to allow then a more widespread use of these implants. Clearly, however, as mentioned above, the present invention is not limited to the use of zygomatic implants, but can be applied to any dental implant designed for implantation into a patient's mouth in a hole made with the aid of a surgical template.

More specifically, the object of the invention is to permit the prediction of the actual final positioning of an implant to be implanted into a patient's mouth with the aid of a surgical template, after the surgical operation has been planned in the way described above and after a surgical template has been made in order to guide the surgeon in making the holes in the planned position, thus allowing the surgeon to decide whether the planned operation can be performed in safe conditions or entails excessive risks.

This and other objects are fully achieved according to the present invention by means of a method comprising the steps specified in the attached independent Claim 1.

Document US 5,556,278 discloses a method for forecasting the actual final position of an implant comprising the steps a) to c) as defined in claim 1 of the present application.

Advantageous embodiments of the invention are described in the dependent claims, the content of which is to be considered as an integral and integrating part of the following description.

Briefly, the invention is based on the idea of providing a surgical template having a bushing positioned and orientated so as to define the axis of the hole to be made in a portion of the patient's cranium (such as the mandible or maxilla, or, if necessary, surrounding regions of the cranium such as the zygoma region); performing a first CT scan on this portion of cranium with the surgical template placed in the same position as that in which it is to be placed during the operation; removing the surgical template from the patient's mouth; performing a second CT scan on the surgical template only; and finally associating the digital model of the surgical template obtained by means of this second CT scan with the digital model of the portion of the patient's cranium obtained by means of the first CT scan, or superimposing it thereon, thus obtaining a final digital model of this portion of the patient's cranium, in which the surgical template with its bushing is shown in the exact position in which it will be placed during the operation. The surgeon can thus check whether the final positioning of the implants coincides with the one initially planned, or whether it is, in any case, such that the operation can be performed without risk (or, in any case, with a certain degree of risk that can be estimated in advance) of causing injury to the patient. This can be achieved, according to a first mode of carrying out the invention, by mounting on the surgical template, once it has been removed from the patient's mouth after the first CT scan, one or more false implants, each having dimensions identical to those of the implant to be implanted, and each being mounted in the respective bushing provided in the surgical template for making the respective hole; and then performing the second CT scan on the surgical template only with the false implants mounted thereon, in such a way that the final digital model of the portion of the patient's cranium shows the false implants in exactly the position in which they will be located after being implanted with the use of the surgical template. According to a second mode of carrying out the invention, the second CT scan of the surgical template only is performed without false implants mounted on it, and the digital models of the implants to be implanted in the patient's mouth are superimposed on the final digital model of the portion of the patient's cranium with the surgical template positioned on it. When a check has been made on the computer to ensure that the surgical template allows to obtain a satisfactory and reliable final positioning of the implants, the surgeon can perform the operation with the assurance that the implants will be positioned at the end of the operation in exactly the position predicted by the prediction method according to the invention, unless, of course, any movements of the surgical template occur during the operation.

Clearly, the prediction method according to the invention will enable the procedure of digital planning of dental implantation operations to become even more efficient and more widely adopted, because, once the surgical template designed by means of the digital planning of the operation has been produced, the surgeon will be able to check whether this surgical template will enable the implants to be positioned in exactly the position planned initially, or in any case in a position such that the operation is feasible and effective. If the outcome of this check is positive, the surgeon can perform the operation more smoothly and safely, using the surgical template as a guide for making the holes for receiving the various implants to be positioned in the patient.

Further characteristics and advantages of the method according to the invention will become clear from the following detailed description which is given purely by way of nonlimiting example with reference to the attached drawings, in which:
Figure 1 is a perspective view of a surgical template intended for use in making a plurality of holes in a patient's mouth (in the maxilla, in this case), each of the holes being intended to receive a respective dental implant (including one of the zygomatic type), together with a false zygomatic implant to be mounted in the respective bushing in the surgical template in order to allow to predict the exact final positioning of this implant in the patient's mouth;
Figure 2 is a perspective view showing the surgical template of Figure 1 with the false zygomatic implant mounted in the respective bushing;
Figure 3 is a perspective view showing a surgical index to be positioned in the patient's mouth, together with the surgical template of Figure 1, in order to keep the surgical template fixed in position;
Figure 4 is a perspective view showing a plaster model of the patient's mouth, in which both the surgical template of Figure 1 and the surgical index of Figure 3 are mounted;
Figure 5 is a picture of the three-dimensional digital model of the assembly formed by the surgical template and the false zygomatic implant of Figures 1 and 2; and
Figure 6 is a picture of the three-dimensional model of the patient's maxilla with the assembly formed by the surgical template and the false zygomatic implant of Figure 5, obtained by the superimposition of the two CT scans.

In the description and subsequent claims, the terms "position" and "positioning" of an implant, or of the hole in which the implant is to be inserted, are used to identify the axis of the implant or of the respective hole, and also, where appropriate, the end point, or apex, of the implant or of the respective hole. Therefore, "predicting the final positioning of an implant" means, for the purposes of the present invention, predicting the position in space of the axis of the implant or of the respective hole. Furthermore, as stated above, the term "implant" is used, for the purposes of the present invention, to denote any dental implant intended to be positioned by means of a surgical template in the patient's mouth, regardless of whether the implant is conventional (that is to say, an implant to be anchored to the bone of the maxilla or mandible), pterygoid (that is to say, an implant to be anchored in the pterygomaxillary process) or zygomatic (that is to say, an implant to be anchored to the zygoma bone). Additionally, terms such as "planned", "plan" and "planning" refer to the digital planning of the operation by means of suitable software tools, and therefore the term "planned position", for instance, means the position determined in advance in the course of the digital planning of the operation. Finally, the term "surgical template" is always used to mean, unless specified otherwise, the surgical template produced on each occasion to enable the holes to be made in the planned position in the patient's mouth.

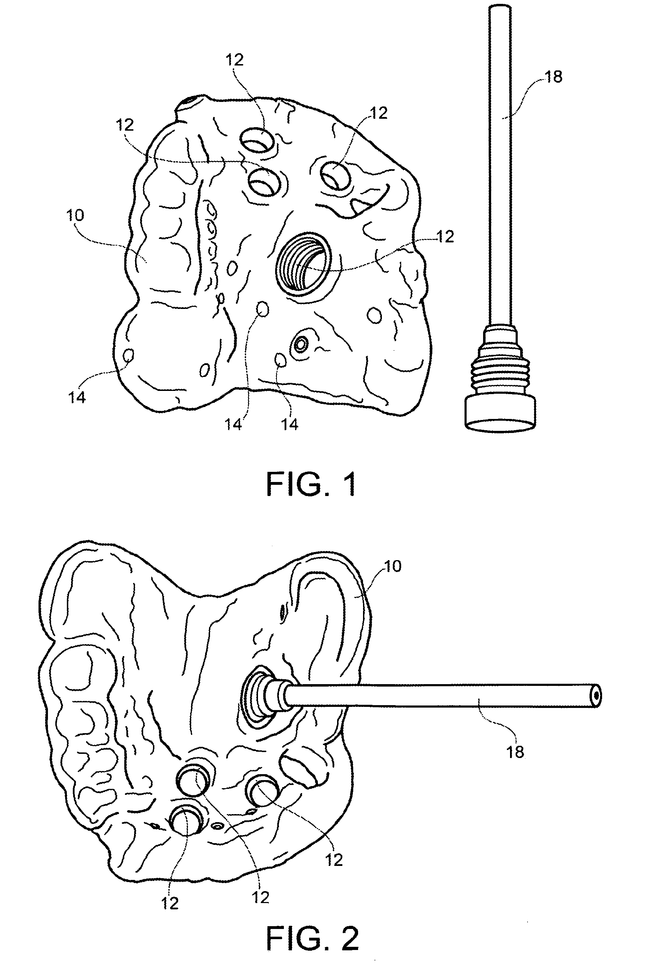

With reference first to Figures 1 and 2, the number 10 generally indicates a surgical template intended for use in dental implantation operations for making one or more holes in the patient's mouth, in each of which holes a respective implant is to be anchored. For this purpose, the surgical template 10 is provided, in a per-se-known manner, with a bushing 12 for each of the holes to be made, the bushing being intended to receive drill guide sleeves for guiding the drills of progressively increasing diameter by means of which the surgeon will make the holes in the patient's mouth (in the maxillary or mandibular bone in the case of conventional dental implants, or in the zygoma bone in the case of zygomatic implants). The bushings 12 are positioned in such a way that, when the surgical template 10 is introduced into the patient's mouth, the axis of each bushing 12 coincides (theoretically at least) with the axis of the respective hole to be made. In the example shown in Figure 1, one of the bushings 12 of the surgical template 10 (specifically, the bushing of greatest diameter) is used for making a hole intended to receive a zygomatic implant.

The surgical template 10 is advantageously produced by a digital planning method such as that described above in the introductory part of the description.

As already mentioned above, if the positioning of the surgical template in the patient's mouth differs from that of the radiographic guide used for the performance of the CT scan required for the digital planning of the operation, then the direction of the axis of each hole defined by the orientation of the respective bushing will inevitably differ from the planned direction, running the risk of causing injury to the patient when the holes are made. In order to eliminate this risk, the invention proposes a method which enables the surgeon to predict, in other words to check before the operation, the exact final positioning of the holes to be made with the surgical template, and thereby to determine whether or not the operation will be feasible, in other words whether or not the actual final position of the holes will coincide with the planned position or in any case will be such that the stability of the implant will be ensured and no injury will be caused to the patient.

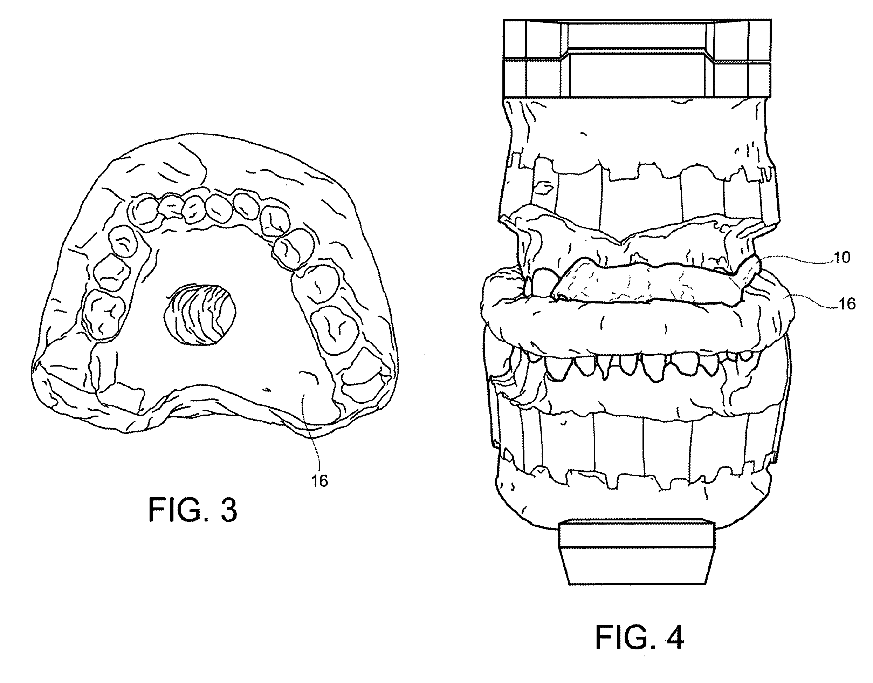

The first step of the method according to the invention is to position radio-opaque markers 14, made of gutta percha or other suitable material, on the surgical template 10, and then to position of the surgical template 10 in the patient's mouth for the acquisition of a CT scan of the portion of the patient's cranium into which the dental implant is to be inserted. In order to stabilize the surgical template 10 in the patient's mouth, provision is advantageously made for the use of a surgical index 16 made of silicone (Figures 3 and 4), which is clamped between the surgical template 10 and the dental arch opposite that into which the implants are to be inserted. The surgical index 16 is coupled on one side to the teeth of the dental arch opposite that into which the implants are to be inserted, and on the opposite side to the surgical template 10.

Once the CT scan of the portion of the patient's cranium with the surgical template 10 positioned on it has been acquired, the surgical template 10 is removed from the patient's mouth and a false implant 18 having the same dimensions as that planned in the digital planning of the operation is inserted into each bushing 12 (or at least into those bushings for which the actual final positioning of the respective implant is to be checked). The false implant 18 is made of a material, such as Arnite (polyethylene terephthalate), which prevents the occurrence of artefacts of the radiographic images. Figure 2 shows the surgical template 10 with the false implant 18 (a zygomatic implant in this example) inserted and secured in the respective bushing 12.

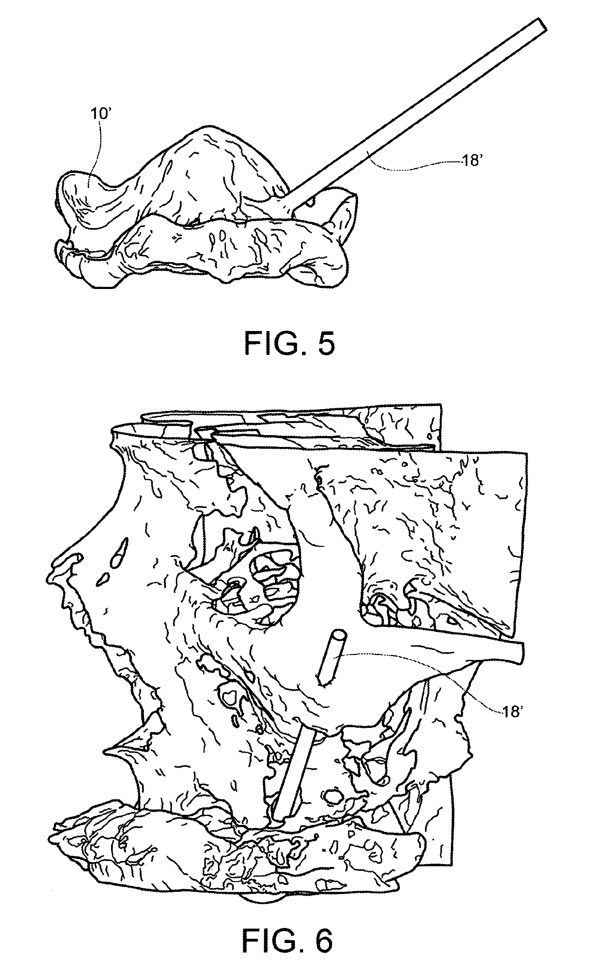

A CT scan is then performed on the surgical template 10 only, fitted with the false implant 18 (or false implants, if the actual final positions of a plurality of implants are to be checked). In this connection, Figure 5 shows a three-dimensional digital model of the assembly formed by the surgical template 10 and the false zygomatic implant 18 obtained on the basis of the last-mentioned CT scan.

At this point, the two CT scans obtained in this way (the CT scan of the portion of the patient's cranium with the surgical template positioned thereon and the CT scan of the surgical template only, including the false implant or implants) are used to obtain a three-dimensional digital model of this portion of the patient's cranium in which it is possible to check the position assumed by each false implant, which will exactly correspond to the final position assumed by the actual implant when it has been inserted into the respective hole made with the aid of the surgical template 10. In this connection, Figure 6 shows an image of the three-dimensional digital model of a portion of the patient's cranium (in the present case, the maxilla and the region of the zygoma and orbits) with the surgical template fitted with a false implant or implants positioned in the patient's mouth. In Figure 6, as in Figure 5, the surgical template and the false implant are indicated 10' and 18' respectively, since these figures show three-dimensional models of the objects instead of the real objects. The three-dimensional model of the portion of the patient's cranium obtained in this way, in which the surgical template is exactly positioned relative to the patient's cranium in the position which it will assume during the operation, and in which the false implant is positioned exactly in the position which it will assume at the end of the operation (provided, of course, that no mistakes are made by the surgeon, owing to displacements of the surgical template for example, when making the hole into which the implant is to be inserted), enables the surgeon to check in advance whether the actual final position of the implant is acceptable and therefore whether the operation is feasible.

Having ascertained the feasibility of the operation, the surgeon can immediately proceed with the operation, using the surgical template 10 for making the holes into which the dental implants are to be inserted.

According to another mode of carrying out the invention, instead of making a false implant and therefore a physical model of the implant whose exact final positioning is to be checked, provision is made to use a three-dimensional digital model of the implant whose exact final positioning is to be checked, and to superimpose this three-dimensional digital model on the three-dimensional digital model of the portion of the patient's cranium with the surgical template positioned thereon. In this case, therefore, the second CT scan of the surgical template only will be performed without false implants fitted thereon. By working on the three-dimensional digital model of the portion of the patient's cranium with the surgical template positioned thereon, and superimposing on this model the three-dimensional digital model of the implant to be implanted into the patient's mouth, the surgeon can thus check the exact final positioning of the implant.

Naturally, the principle of the invention remaining the same, the modes of implementation may be varied widely with respect to those described and illustrated purely by way of nonlimiting example, without thereby departing from the scope of the invention as defined in the attached claims.

## Claims

1. Method for predicting the actual final positioning of a dental implant to be implanted in a patient's mouth in a hole to be made in a portion of the patient's cranium with the aid of a surgical template (10), the method comprising the steps of:
a) providing a surgical template (10) having a bushing (12) positioned and orientated so as to define the axis of the aforesaid hole, said bushing (12) being adapted to receive drill guide sleeves with which drills of progressively increasing diameter are to be guided for the making of the aforesaid hole;
b) performing a CT scan of said portion of the patient's cranium with the surgical template (10) positioned thereon;
c) removing the surgical template (10) from the patient's mouth;
d) performing a CT scan on the surgical template (10) only; and
e) generating, from the CT scans obtained in steps b) and d), a three-dimensional digital model of said portion of the patient's cranium, in which the surgical template (10), and therefore the axis of the aforesaid hole, are placed in the same position as that in which they were placed in step b).

2. Method according to Claim 1, wherein step a) further comprises the operation of providing a series of radio-opaque markers (14) on the surgical template (10).

3. Method according to Claim 1 or 2, wherein step b) is carried out with a surgical index (16) additionally positioned in the patient's mouth, the index being interposed between the surgical template (10) and the dental arch opposite that into which the dental implant is to be inserted.

4. Method according to any of the preceding claims, wherein the dental implant is a zygomatic implant.

5. Method according to any of the preceding claims, further comprising, between steps c) and d), the step c') of fitting into the bushing (12) a false dental implant (18) having dimensions identical to those of the dental implant whose actual final positioning is to be checked, step d) providing for the performance of a CT scan of the surgical template (10) together with the false dental implant (18) fitted thereon in step c').

6. Method according to any of Claims 1 to 4, further comprising, after step e), the step f) of superimposing, on the three-dimensional digital model of said portion of the patient's cranium with the surgical template (10) positioned thereon, a digital model of the dental implant whose final positioning is to be checked.

## Patentansprüche

1. Verfahren zum Vorhersagen der tatsächlichen endgültigen Positionierung eines in eines Patienten Mund in einem Loch, das in einem Abschnitt des Patienten Schädels mit der Hilfe einer chirurgischen Schablone (10) auszubilden ist, zu implantierenden Zahnimplantats, mit den Schritten:
a) Vorsehen einer chirurgischen Schablone (10) mit einer Buchse (12), die so positioniert und orientiert ist, dass sie die Achse des zuvor genannten Lochs definiert, und dazu angepasst ist, Bohrführungshülsen aufzunehmen, mit denen Bohrer fortschreitend zunehmenden Durchmessers für das Ausbilden des zuvor genannten Lochs zu führen sind;
b) Durchführen eines CT-Scans besagten Abschnitts des Patienten Schädels mit der darauf positionierten chirurgischen Schablone (10);
c) Entfernen der chirurgischen Schablone (10) aus des Patienten Mund;
d) Durchführen eines CT-Scans nur auf der chirurgischen Schablone (10); und
e) Erzeugen eines dreidimensionalen digitalen Modells besagten Abschnitts des Patienten Schädels aus den CT-Scans, die in Schritten b) und d) erhalten werden, in dem die chirurgische Schablone (10) und daher die Achse des zuvor genannten Lochs in derselben Position wie jener, in der sie in Schritt b) platziert waren, platziert sind.

2. Verfahren nach Anspruch 1, bei dem Schritt a) ferner den Vorgang von Vorsehen einer Reihe strahlenundurchlässiger Markierungen (14) auf der chirurgischen Schablone (10) aufweist.

3. Verfahren nach Anspruch 1 oder 2, bei dem Schritt b) mit einem Bissregistrat (16) ausgeführt wird, das zusätzlich in des Patienten Mund positioniert ist, bei dem das Registrat zwischen der chirurgischen Schablone (10) und dem Zahnbogen gegenüber jenem, in den das Zahnimplantat einzusetzen ist, eingefügt ist.

4. Verfahren nach einem der vorhergehenden Ansprüche, bei dem das Zahnimplantat ein zygomatisches Implantat ist.

5. Verfahren nach einem der vorhergehenden Ansprüche, ferner mit dem Schritt c') von Einsetzen eines falschen Zahnimplantats (18) in die Buchse (12), das zu jenen des Zahnimplantats, dessen tatsächliche endgültige Positionierung zu überprüfen ist, identische Dimensionen aufweist, zwischen Schritten c) und d), bei dem Schritt d) die Durchführung eines CT-Scans der chirurgischen Schablone (10) zusammen mit dem in Schritt c') darauf aufgesetzten falschen Zahnimplantat (18) vorsieht.

6. Verfahren nach einem der Ansprüche 1 bis 4, ferner mit dem Schritt f) von Überlagern eines digitalen Modells des Zahnimplantats, dessen endgültige Positionierung zu überprüfen ist, auf dem dreidimensionalen digitalen Modell besagten Abschnitts des Patienten Schädels mit der darauf positionierten chirurgischen Schablone (10) nach Schritt e).

## Revendications

1. Procédé pour calculer la position finale réelle d'un implant dentaire à implanter dans la bouche d'un patient dans un trou à forer dans une partie du crâne du patient à l'aide d'un guide chirurgical (10), le procédé comprenant les étapes de :
a) fourniture d'un guide chirurgical (10) ayant des bagues d'arrêt (12) positionnées et orientées de manière à définir l'axe du trou susmentionné, lesdites bagues d'arrêt (12) étant configurées pour recevoir des douilles guide-mèches avec lesquelles les mèches au diamètre progressivement croissant doivent être guidées pour le forage du trou susmentionné ;
b) réalisation d'une TDM de ladite partie du crâne du patient, le guide chirurgical (10) étant positionné dessus ;
c) retrait du guide chirurgical (10) de la bouche du patient ;
d) réalisation d'une TDM sur le guide chirurgical (10) uniquement ; et
e) génération, à partir des clichés TDM obtenus aux étapes b) et d), d'un modèle numérique tridimensionnel de ladite partie du crâne du patient, dans lequel le guide chirurgical (10), et par conséquent l'axe du trou susmentionné, sont placés dans la même position que celle dans laquelle ils étaient placés à l'étape b).

2. Procédé selon la revendication 1, dans lequel l'étape a) comprend en outre l'opération de fourniture d'une série de repères radio-opaques (14) sur le guide chirurgical (10).

3. Procédé selon la revendication 1 ou la revendication 2, dans lequel l'étape b) est mise en oeuvre avec un moulage chirurgical (16) en outre positionné dans la bouche du patient, le moulage étant interposé entre le guide chirurgical (10) et l'arcade dentaire à l'opposé de celle dans laquelle l'implant dentaire doit être inséré.

4. Procédé selon l'une quelconque des revendications précédentes, dans lequel l'implant dentaire est un implant zygomatique.

5. Procédé selon l'une quelconque des revendications précédentes, comprenant en outre, entre les étapes c) et d), l'étape c') de fixation dans les bagues d'arrêt (12) d'un implant dentaire artificiel (18) ayant des dimensions identiques à celles de l'implant dentaire dont la position finale réelle doit être vérifiée, l'étape d) permettant la réalisation d'une TDM du guide chirurgical (10) conjointement avec l'implant dentaire artificiel (18) fixé dessus à l'étape c').

6. Procédé selon l'une quelconque des revendications 1 à 4, comprenant en outre, après l'étape e), l'étape f) de superposition, sur le modèle numérique tridimensionnel de ladite partie du crâne du patient, le guide chirurgical (10) étant positionné dessus, d'un modèle numérique de l'implant dentaire dont la position finale doit être vérifiée.
